# EUROPÄISCHE PATENTSCHRIFT

(11) **EP 4 522 064 B1**
(45) Veröffentlichungstag und Bekanntmachung des Hinweises auf die Patenterteilung: **24.06.2026**
(21) Anmeldenummer: 24739491.9
(22) Anmeldetag: 03.07.2024
(51) Int. Cl.: A61B 90/30, F21V 29/70, F21V 15/01, F21V 19/00, F21W 131/202, F21Y 115/10

(54) **MEDIZINISCHE LEUCHTE**
MEDICAL LIGHT
LAMPE MÉDICALE

(30) Priorität: 28.07.2023 EP 23188307
(43) Veröffentlichungstag der Anmeldung: 19.03.2025
(73) Patentinhaber: KaVo Dental GmbH, 88400 Biberach an der Riss (DE)
(72) Erfinder: WEILER, Evelyn, 88456 Ingoldingen (DE); ZAENGEL, Fabian, 89257 Illertissen (DE); RIEGER, Kerstin, 89195 Staig (DE); HACKEL, André, 88400 Biberach (DE)
(74) Vertreter: Thun, Clemens
(86) Internationale Anmeldenummer: PCT/EP2024/068685
(87) Internationale Veröffentlichungsnummer: WO 2025/026635

(56) Entgegenhaltungen:
- EP-A1- 3 321 571
- EP-A1- 3 671 035
- US-A1- 2003 036 031
- US-A1- 2010 210 918
- US-B1- 11 608 961

## Beschreibung

Die vorliegende Erfindung betrifft eine medizinische Leuchte, insbesondere eine zahnärztliche Behandlungsleuchte, die für das Ausleuchten eines Operationsfelds vorgesehen ist.

Um eine optimale medizinische Behandlung gewährleisten zu können, ist es im Fall von Operationen oder zahnärztlichen Behandlungen bzw. Untersuchungen unerlässlich, die Behandlungsstelle geeignet auszuleuchten. Dabei sind verschiedene Rahmenbedingungen einerseits zum Schutz des Patienten, aber andererseits auch zur Optimierung der Ausleuchtung zu beachten, die Eingang in verschiedene Normen gefunden haben und bspw. die Form des Lichtfeldes, einen bestimmten Mindestwert des Farbwiedergabeindexes der Beleuchtung sowie einen Mindestwert der Beleuchtungsintensität vorschreiben.

Eine Leuchte, welche diese Anforderungen erfüllt, ist bspw. aus der EP 2 469 158 A2 der Anmelderin bekannt. Dieses Dokument beschreibt eine Dentalleuchte, bei welcher zur Erzielung der gewünschten Ausleuchtung fünf getrennte Beleuchtungseinheiten zum Einsatz kommen, die jeweils LED-Lichtquellen sowie zugehörige Optiken aufweisen. Diese fünf Beleuchtungseinheiten sind räumlich verteilt in einem Leuchtengehäuse integriert, wobei die LED-Lichtquellen zur Gewährleistung einer überlagernden Projektion in einer Objektebene auf einem gemeinsamen Träger platziert und entsprechend ausgerichtet sind. Hierdurch wird eine schattenfreie, möglichst homogene Ausleuchtung des Operationsfelds erzielt, die selbst dann beibehalten wird, wenn das Licht einzelner Beleuchtungseinheiten bspw. durch den Arzt abgeschattet wird. Die mehreren Beleuchtungseinheiten sind hierbei wie erwähnt auf einem gemeinsamen Träger angeordnet, der eine geeignete Ausrichtung der Beleuchtungseinheiten zueinander gewährleistet und thermisch an das Leuchtengehäuse angekoppelt ist. Hierdurch kann eine relativ homogene Wärmeverteilung am Leuchtengehäuse erzielt werden, was insofern von Vorteil ist, als zur Vermeidung von Irritationen bei der Verwendung der Leuchte oder allgemein von Medizinprodukten es unerlässlich ist, eine gleichmäßige und nicht zu hohe Oberflächentemperatur zu realisieren.

Bei der im Stand der Technik bekannten Leuchte ist zum Erzielen eines optimalen Beleuchtungsergebnisses unerlässlich, die mehreren Beleuchtungseinheiten exakt aufeinander abgestimmt auszurichten. Da dies mit einem relativ hohen Aufwand verbunden ist, wäre es grundsätzlich von Vorteil, wenn die Erzeugung des Lichts auf einen kleineren Bereich konzentriert werden könnte. In diesem Fall könnte dann das Licht einfacher und effizienter kontrolliert bzw. beeinflusst werden, so dass mit geringerem Aufwand eine vergleichbare oder sogar noch höhere Qualität der Beleuchtung erzielt wird.

Die Konzentration der Lichterzeugung auf einen kleineren Bereich führt allerdings zu dem Problem, dass im Vergleich zu der verteilten Anordnung der Leuchtmittel im Stand der Technik lokal eine höhere Wärmeerzeugung stattfindet und somit die Gefahr erhöht wird, dass an dem Leuchtengehäuse punktuelle sog. Hotspots entstehen. Abgesehen davon, dass hierdurch die Gefahr einer Beschädigung elektronischer Komponenten erhöht wird, stellen derartige Hotspots auch eine Gefährdung für den Patienten oder den Arzt dar.

Eine vergleichbare Problematik ergibt sich auch bei Leuchten, bei denen das Licht einer kompakten LED-Lichtquelle mit Hilfe eines topfartigen Reflektors abgegeben wird. Eine derartige sog. Monoreflektor-Operationsleuchte, welche die Merkmale des Oberbegriffs des Anspruchs 1 aufweist, ist aus der EP 3 321 571 A1 bekannt.

Eine Anordnung zur Lichtabgabe, bei der eine Lichtquelle in Form eines auf einer Platine angeordneten LED-Leuchtmittels in einem topfartigen Gehäuse angeordnet ist, welches eine zur Lichtabgabe vorgesehene Lichtaustrittsöffnung bildet und einen Bodenbereich zur flächigen Lagerung der Platine sowie einen sich von dem Bodenbereich zur Lichtaustrittsöffnung umlaufend erstreckenden Wandbereich aufweist, ist in der US 2003/036031 A1 beschrieben. Diese Anordnung ist allerdings Bestandteil eines zahnmedizinischen Instruments zum Aushärten von Dentalfüllungen, nicht jedoch einer Leuchte, welche zu Beleuchtungszwecken genutzt wird.

Der vorliegenden Erfindung liegt deshalb die Aufgabenstellung zugrunde, eine medizinische, insbesondere eine zahnmedizinische Behandlungsleuchte zu Verfügung zu stellen, welche derart ausgeführt ist, dass die während des Betriebs auftretende Wärme möglichst homogen verteilt über das Gehäuse abgeführt werden kann.

Die Aufgabe wird durch eine Leuchte, welche die Merkmale des Anspruchs 1 aufweist, gelöst, vorteilhafte Weiterbildungen der Erfindung sind Gegenstand der abhängigen Ansprüche.

Die erfindungsgemäße Lösung beruht auf einer speziellen Ausgestaltung des die Leuchtmittel aufnehmenden Leuchtengehäuses, welches derart ausgeführt ist, dass in dem Gehäuse erzeugte Wärme äußerst effizient verteilt und großflächig an die Umgebung abgeführt werden kann. Damit eignet sich das erfindungsgemäße Konzept insbesondere für Leuchten, bei denen nur wenige, auf einen kleinen Bereich konzentrierte Leuchtmittel für die Lichterzeugung zum Einsatz kommen. Grundsätzlich kann die erfindungsgemäße Lösung allerdings selbstverständlich auch in solchen Fällen zum Einsatz kommen, bei denen die Leuchtmittel über einen größeren Bereich verteilt angeordnet sind.

Dabei ist erfindungsgemäß vorgesehen, dass das topfartig ausgebildete Leuchtengehäuse, auf dessen Bodenbereich die Anordnung zumindest einer LED-Platine vorgesehen ist, mehrschichtig ausgebildet ist. Insbesondere ist das Gehäuse erfindungsgemäß durch eine aus Kunststoff bestehende Außenschale sowie eine aus Metall bestehende, an der Innenseite der Außenschale anliegende Innenschale gebildet, wobei die LED-Platine in flächigem Wärmekontakt mit der Innenschale ist und diese sich über den Bodenbereich des Leuchtengehäuses bis in den Wandbereich hinein erstreckt.

Gemäß der vorliegenden Erfindung wird also eine medizinische Leuchte, insbesondere eine zahnärztliche Behandlungsleuchte für das Ausleuchten eines Operationsfeldes vorgeschlagen, die aufweist:
- eine Lichtquelle, welche zumindest ein auf einer Platine angeordnetes LED-Leuchtmittel aufweist, sowie
- ein topfartiges Gehäuse zur Aufnahme der Lichtquelle, welches eine zur Lichtabgabe vorgesehene Lichtaustrittsöffnung bildet;
wobei das Gehäuse einen Bodenbereich zur flächigen Lagerung der Platine sowie einen sich von dem Bodenbereich zur Lichtaustrittsöffnung umlaufend erstreckenden Wandbereich aufweist, und wobei erfindungsgemäß das Gehäuse durch eine aus Kunststoff bestehende Außenschale sowie eine aus Metall bestehende, an der Innenseite der Außenschale anliegende Innenschale gebildet wird und die Platine in flächigem Wärmekontakt mit der Innenschale ist, welche sich über den Bodenbereich in den Wandbereich hinein erstreckt.

Es hat sich herausgestellt, dass diese zweischichtige oder zweilagige Ausgestaltung des Leuchtengehäuses in äußerst effizienter Weise dazu führt, dass die von dem Leuchtmittel erzeugte Wärme gleichmäßig verteilt und letztendlich nahezu über die gesamte Außenfläche des Gehäuses homogen an die Umgebung abgegeben werden kann. Insbesondere ist die hierbei erzielbare Wärmeverteilung derart effizient, dass selbst bei einem längeren Betrieb bei maximaler Leistung der Leuchte an deren Oberfläche keine Temperaturen auftreten, die ggf. von einem Patienten oder einem Arzt bzw. generell von einem Benutzer der Leuchte als unangenehm empfunden werden. Dennoch ist eine für den Betrieb der Leuchte unerlässliche zuverlässige Ableitung der Wärme gewährleistet, was insofern überraschend ist, als die Außenfläche des erfindungsgemäßen Leuchtengehäuses aus Kunststoff besteht, also aus einem Material, welches an sich keine gute Wärmeleitfähigkeit aufweist. Die erfindungsgemäße Nutzung der Innenschale führt allerdings dazu, dass diese an sich nachteilige Eigenschaft der Außenschale nicht zum Tragen kommt. Somit kann also die Außenseite des Leuchtengehäuses vollständig aus Kunststoff, als einem elektrisch isolierenden Material gebildet sein, was dann wiederum zu Vorteilen in der Handhabung und der Sicherheit der Leuchte führt. Im Vergleich zu einer einfachen Wärmesenke, also beispielsweise einem Metallblock, der die Wärme des LED-Leuchtmittels aufnimmt, unterscheidet sich hierbei das erfindungsgemäße Konzept darin, dass die Innenschale tatsächlicher, funktionaler Bestandteil des Leuchtengehäuses ist und - wie nachfolgend noch näher beschrieben - auch zusätzliche Gehäusefunktionen erfüllen kann.

Dabei kann vorgesehen sein, dass die Außenschale im Wandbereich des Gehäuses die Innenschale überragt und sich bis zur Lichtaustrittsöffnung hin erstreckt. Insbesondere kann hierbei die Außenschale in dem der Lichtaustrittsöffnung zugewandten Wandbereich Befestigungsmittel zur Halterung einer die Lichtaustrittsöffnung schließenden lichtdurchlässigen Abdeckung und/oder optischer Elemente zur Beeinflussung des von der Lichtquelle abgegebenen Lichts aufweisen.

Die Außenschale, also der aus Kunststoff bestehende Bereich des Leuchtengehäuses kann dabei ferner ein oder mehrere Griffelemente bilden, über welche die Leuchte in eine bestimmte Position gebracht oder ausgerichtet werden kann. Auch hier ist von Vorteil, dass diese Komponenten des Gehäuses aus Kunststoff, also einem elektrisch isolierenden Material bestehen.

Die Innenschale wiederum kann vorzugsweise im Bodenbereich Strukturelemente zur definierten Positionierung der Platine aufweisen, wobei diese Strukturelemente insbesondere Stifte oder Stege bilden, welche in der Platine ausgebildete Zentrierungsöffnungen durchgreifen. Hierdurch wird eine exakte Anordnung des Leuchtmittels gewährleistet, was insofern von Vorteil ist, als auf diesem Wege auch eine geeignete Positionierung der Leuchtmittel in Bezug auf die weiteren optischen Elemente erfolgt. Hierdurch ist gewährleistet, dass das Licht in geeigneter Weise beeinflusst werden kann, um die gewünschte homogene und gleichmäßige Ausleuchtung des zu behandelnden Bereichs zu ermöglichen. Dabei ist besondere bevorzugt vorgesehen, dass die Platine zwei Zentrierungsöffnungen aufweist, wobei eine der Zentrierungsöffnungen einen auf die Außenkontur des Stifts oder Stegs angepassten Querschnitt aufweist und die andere Zentrierungsöffnung durch ein Langloch gebildet ist, welches eine Relativverschiebung zwischen Platine und Innenschale ermöglicht. Hierdurch kann das Auftreten von Spannungen aufgrund unterschiedlicher temperaturbedingter Ausdehnungen vermieden werden. Die Befestigung der Platine an der Innenschale kann dann mithilfe eines rahmenartigen Klemmelements erfolgen, welches die Platine federnd auf die Innenschale drückt, wobei insbesondere das Klemmelement mittels einer Schraubhalterung an der Innenschale befestigt ist und vorzugsweise die Strukturelemente Schraubkanäle zur Befestigung des Klemmelements bilden.

Das LED-Leuchtmittel ist vorzugsweise zentral auf der Platine angeordnet und beansprucht etwa lediglich 1% der Fläche der Platine. Dabei ist besonders bevorzugt vorgesehen, dass das LED-Leuchtmittel durch zwei Reihenschaltungen von mehreren, insbesondere von jeweils drei LEDs gebildet ist.

Eine weitere Verbesserung der Übertragung der Wärme von dem LED-Leuchtmittel auf die Innenschale des Leuchtengehäuses kann ferner dadurch erzielt werden, dass die Platine an ihrer dem LED-Leuchtmittel gegenüberliegenden Rückseite sog. Wärmeleitpads aufweist, welche sich strahlen- bzw. fächerförmig ausgehend vom Zentrum der Platine nach außen erstrecken und mindestens 80% der Platinenfläche einnehmen. Diese Wärmeleitpads bilden dann elektrisch voneinander isolierte Bereiche, die vorzugsweise jeweils über Durchkontaktierungen mit Anschlüssen des LED-Leuchtmittels gekoppelt sind.

Gemäß einer besonders bevorzugten Ausführungsform kann ferner vorgesehen sein, dass die Innenschale ein Lagergehäuse zur Halterung der Leuchte an einem Tragarm aufweist, wobei das Lagergehäuse insbesondere zur drehbaren Aufnahme des Tragarms ausgebildet ist. Dieser Aspekt unterstreicht nochmals den Gedanken, dass die Innenschale tatsächlicher Bestandteil des Leuchtengehäuses ist, da auf diesem Wege eine dauerhaft zuverlässige Halterung der Leuchte an einem Tragarm bzw. generell an entsprechenden Tragelementen gewährleistet ist.

Die Wärmeleitfähigkeit des Materials der Innenschale ist etwa um den Faktor 100 größer als die Wärmeleitfähigkeit des Materials der Außenschale. Insbesondere kann die Innenschale aus Aluminium oder Magnesium bestehen, wobei es sich besonders bevorzugt um ein Aluminium- oder Magnesiumdruckgussteil handelt.

Ein effizientes Zusammenwirken zwischen Innenschale und Außenschale im Hinblick auf die Wärmeübertragung erfordert, dass beide Komponenten flächig aneinander liegen und insbesondere keine thermisch isolierenden Lufteinschlüsse bestehen. Es hat sich deshalb als besonders vorteilhaft herausgestellt, wenn die Außenschale durch Aufspritzen von Kunststoffmaterial auf die Innenschale gebildet wird, so dass diese unmittelbar auf der Innenschale anliegt. In diesem Fall wird zwar aufgrund der unterschiedlichen Materialien keine feste Verbindung zwischen beiden Komponenten erzielt, dennoch findet eine ausreichende Wärmeübertragung von Innenschale zu Außenschale statt. Ferner ist von Vorteil, wenn die Außenschale einen Randbereich der Innenschale zumindest teilweise übergreift, so dass trotz des Fehlens einer stoffschlüssigen Verbindung beide Schalen fest miteinander verbunden sind.

Letztendlich wird durch die verschiedenen Maßnahmen also ein Leuchtengehäuse für eine medizinische Leuchte bereitgestellt, welches selbst im Falle einer starken lokalen Konzentration der Wärmeentwicklung eine gleichmäßige, großflächige und homogene Wärmeabgabe an die Umgebung erzielt.

Nachfolgend soll die Erfindung anhand der beiliegenden Zeichnung näher erläutert werden. Es zeigen:
- Fig. 1: ein Ausführungsbeispiel einer erfindungsgemäßen Leuchte, die an einem Trägersystem eines zahnärztlichen Behandlungsplatzes angeordnet ist;
- Fig. 2: eine vergrößerte Ansicht der Leuchte von Figur 1;
- Fig. 3: eine Ansicht des Innenraums des Leuchtengehäuses mit der darin angeordneten LED-Platine;
- Fig. 4: eine Schnittdarstellung der Ansicht von Fig. 3;
- Fig. 5: eine Darstellung der Vorderseite der LED-Platine mit dem LED-Leuchtmittel;
- Fig. 6: eine Darstellung der Rückseite der LED-Platine;
- Fig. 7: eine weitere Ansicht des Innenraums der Leuchte mit dem darin angeordneten Klemmelement zur Halterung der LED-Platine;
- Fig. 8: eine Schnittdarstellung der Ansicht von Fig. 7; und
- Fig. 9: eine schematische Darstellung zur Verdeutlichung der mithilfe der erfindungsgemäßen Lösung erzielbaren Wärmeabfuhr.

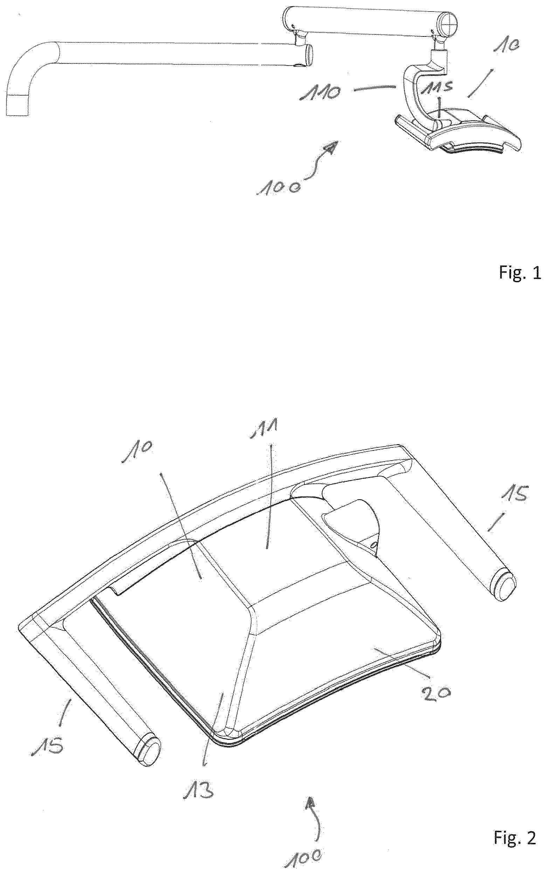

Figur 1 zeigt zunächst in Übersicht eine erfindungsgemäße, mit dem Bezugszeichen 100 versehene dentalmedizinische Leuchte, die an einer Tragstruktur 110 angeordnet ist, welche beispielsweise Bestandteil eines nicht dargestellten zahnärztlichen Behandlungsplatzes ist. Sowohl die Tragstruktur 110 als auch die nachfolgend noch näher beschriebene Halterung der Leuchte 100 an dieser Tragstruktur 110 kann mehrere Gelenke aufweisen, über welche ein einfaches Verstellen der Position der Leuchte 100 sowie eine geeignete Ausrichtung ermöglicht wird, wie dies von Dentalleuchten bekannt ist.

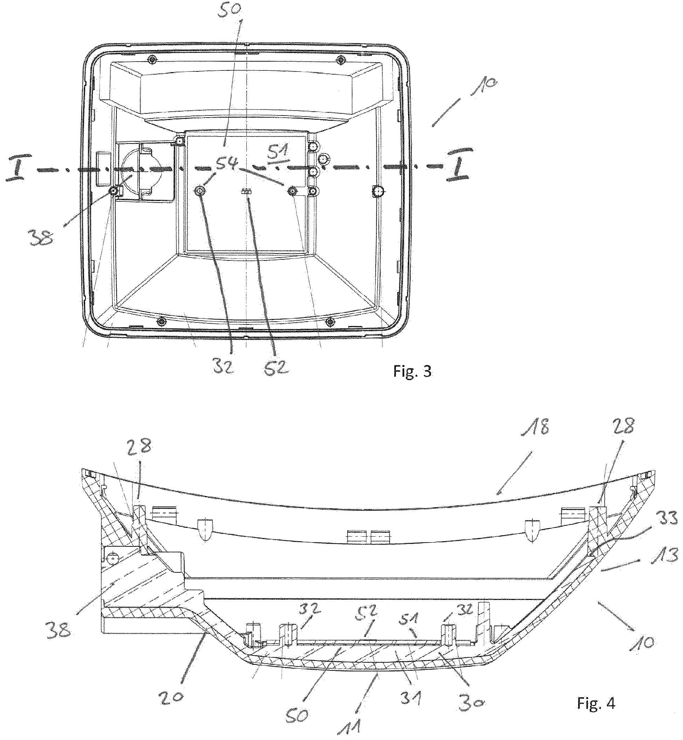

Das Leuchtengehäuse 10, welches in Figur 2 näher erkennbar ist, weist grundsätzlich eine topfartige Form auf und bildet hierbei einen die Rückseite der Leuchte 100 bildenden Bodenbereich 11 sowie einen umlaufenden Wandbereich 13, der eine Lichtaustrittsöffnung 18 (siehe beispielsweise Figur 4) begrenzt. Im vorliegenden Ausführungsbeispiel ist das Leuchtengehäuse 10 etwa pyramidenstumpfartig ausgeführt, sodass sich für die Lichtaustrittsöffnung 18 eine im Wesentlichen quadratische Form ergibt. Grundsätzlich wäre allerdings auch beispielsweise eine kegelstumpfartige Form für das Gehäuse 10 oder eine ovale Form für die Lichtaustrittsöffnung 18 denkbar. Entscheidend ist, dass ein in etwa ebener Bodenbereich 11 gebildet ist, welcher der nachfolgend noch näher beschriebenen Lagerung des Leuchtmittels dient, wobei sich ausgehend von dem Bodenbereich 11 geschlossen umlaufend der Wandbereich 13 bis zur Lichtaustrittsöffnung 18 hin erstreckt.

Wie ferner Figur 2 zeigt, sind im vorliegenden Fall an dem Gehäuse 10 zwei einander gegenüberliegend angeordnete Griffelemente 15 vorgesehen, welche ein Greifen der Leuchte 100 und somit ein Positionieren bzw. Ausrichten ermöglichen. Die bereits erwähnten Gelenke des Lagerungssystems sind dann derart ausgeführt, dass die Leuchte 100 in der eingestellten Position und Ausrichtung verharrt.

Die in Figur 2 erkennbaren Bestandteile des Leuchtengehäuses 10, also dessen Außenflächen sowie vorzugsweise auch die einstückig daran angeordneten Griffelemente 15 werden durch eine erste Komponente des Gehäuses 10 gebildet, die nachfolgend auch als Außenschale 20 bezeichnet wird. Diese - vorzugsweise einstückige - Außenschale 20 besteht aus Kunststoff, also einem elektrisch isolierenden Material, wodurch ein Berühren der Leuchte 100 ohne die Gefahr eines elektrischen Schlags problemlos ermöglicht ist. Ferner für die Verwendung von Kunststoff im Vergleich zu Metall in der Regel zu einem angenehmeren Griffgefühl.

Die dargestellte Form inklusive der Griffelemente 15 des Gehäuses 10 lässt sich bei der Verwendung von Kunststoffmaterial in einfacher Weise im Spritzgussverfahren herstellen, wobei die entsprechenden Strukturen mit hoher Genauigkeit realisiert werden können, sodass das Leuchtengehäuse 10 mit einer qualitativ hochwertigen Außenfläche realisiert werden kann. Gleichzeitig besteht allerdings das Problem, dass Kunststoff an sich schlecht wärmeleitfähig ist und somit die während des Betriebs der Leuchte 100 entstehende Wärme durch Kunststoffgehäuse in der Regel eher schlecht an die Umgebung abgeleitet werden kann. Insbesondere besteht das Problem, dass das Kunststoffmaterial nicht in der Lage ist, die konzentriert in einzelnen Bereichen auftretende Wärme effizient und gleichmäßig zu verteilen.

Zur Lösung dieses Problems und gemäß der vorliegenden Erfindung ist deshalb vorgesehen, das Gehäuse 10 zweilagig, also zusätzlich zur Außenschale 20 mit einer - wiederum vorzugsweise einstückigen - Innenschale 30 auszugestalten, welche deutlich andere thermische Eigenschaften hat und mit deren Hilfe die Nachteile des Kunststoffmaterials der Außenschale 20 kompensiert werden. Dieses Konzept soll nachfolgend anhand der Figuren 3 und 4 näher erläutert werden.

Figur 3 zeigt hierbei den Innenbereich des Leuchtengehäuses 10, welches in den Figuren durch eine nicht näher dargestellte, lichtdurchlässige Abdeckung geschlossen ist. Figur 4 wiederum ist eine Schnittdarstellung entlang der Achse I-I in Figur 3.

Erfindungsgemäß ist also vorgesehen, dass an der Innenseite der Außenschale 20, die wie bereits erwähnt aus Kunststoff besteht, eine wiederum topfartig ausgebildete Innenschale 30 angeordnet ist. Diese Innenschale 30, welche wie nachfolgend erläutert aus Metall besteht, bildet also ebenfalls einen dem Bodenbereich 11 des Leuchtengehäuses 10 entsprechenden Bodenbereich 31, von dem sich ausgehend in Richtung der Lichtaustrittsöffnung 18 ein umlaufender Wandbereich 33 erstreckt. Innenschale 30 und Außenschale 20 liegen flächig aneinander an, sind allerdings aufgrund der verschiedenen Materialien nicht stoffschlüssig miteinander verbunden.

Die nun tatsächlich ebene Oberfläche des Bodenbereichs 31 der Innenschale 30 dient der Lagerung einer Lichtquelle 50, die im dargestellten Ausführungsbeispiel durch eine quadratische Platine 51 mit einem zentral darauf angeordneten LED-Leuchtmittel 52 gebildet ist. Bei dem LED-Leuchtmittel 52 kann es sich um ein oder mehrere LEDs oder eine Kombination mehrerer LEDs handeln. Wie in den Figuren erkennbar, ist vorzugsweise vorgesehen, dass das LED-Leuchtmittel 52 lokal auf den zentralen Bereich der Platine 51 beschränkt ist, also nur geringe Abmessungen aufweist. Zwar wäre es auch denkbar, die Platine 51 großflächig mit LEDs zu bestücken, die in den Figuren dargestellte Variante bringt allerdings den Vorteil mit sich, dass das Licht der hier zum Einsatz kommenden, quasi punktartigen Lichtquelle besser und effizienter durch geeignete optische Mittel beeinflusst werden kann. Letztendlich kann hierdurch die Qualität der Beleuchtung verbessert werden, wobei durch den Einsatz geeigneter optischer Mittel insbesondere die Möglichkeit besteht, den Operations- oder Untersuchungsbereich homogen und schattenfrei zu beleuchten.

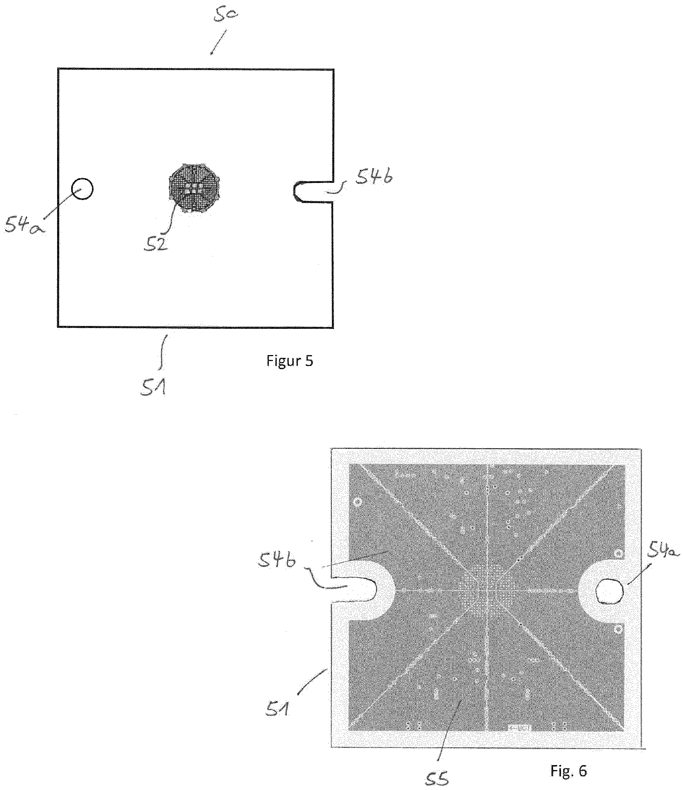

Ein bevorzugtes Ausführungsbeispiel zur Realisierung des LED-Leuchtmittel 52 ist in Figur 5 dargestellt. In diesem Fall besteht das LED-Leuchtmittel 52 aus zwei parallel zueinander angeordneten Reihenschaltungen von jeweils drei LEDs 53a und 53b, so dass durch wahlweise Aktivierung der beiden LED-Gruppen 53a, 53b zwischen verschiedenen Lichtmodi in Abhängigkeit der jeweiligen medizinischen Indikation gewählt werden kann.

Beide LED-Gruppen beanspruchen gemeinsam maximal etwa 1% der Gesamtfläche der LED-Platine 51, was verdeutlicht, dass es sich tatsächlich um eine extrem kompakte, nahezu punktförmige Lichtquelle handelt, deren Licht effizient zur Beleuchtung eines medizinischen Arbeitsbereichs geformt werden kann. Jede der drei jeweils in Reihe geschalteten LEDs weist vier elektrische Kontaktfahnen (Kathoden und Anoden) auf, die gleichzeitig auch thermische Wärmeleiter bilden. Um die im geschlossenen Gehäuse als Wärmehotspot auftretende Wärme möglichst schnell abzusenken und gleichmäßig an das Gehäuse 10 zur weiteren Wärmeabgabe zu verteilen, werden die elektrischen Anschlussfahnen auf die metallisch ausgebildete Rückseite der Platine 51 durchkontaktiert, so dass sie mit dort vorgesehenen Wärmeleitpads 55 in Verbindung stehen.

Für ein effizientes Zusammenwirken zwischen LED-Leuchtmittel 52 und - nicht dargestellter - Optik ist erforderlich, dass das LED-Leuchtmittel 52 exakt innerhalb des Leuchtengehäuses 10 positioniert ist. Hierzu sind an der Oberseite des Bodenbereichs 31 der Innenschale 30 Strukturelemente in Form von Stiften bzw. Stegen 32 ausgebildet, die entsprechende Öffnungen 54a, 54b in der Platine 51 durchgreifen. Hierdurch ist eine definierte Lagerung der Platine 51 in dem Gehäuse 10 und damit letztendlich der eigentlichen Lichtquelle 50 gewährleistet.

Wie hierbei in Figur 6 erkennbar ist, kann gemäß eine bevorzugten Ausführungsform eine der beiden Öffnungen der Platine 51 als geschlossenes Loch 54a ausgeführt sein, dessen Querschnitt an die Außenkontur des Stegs 32 angepasst ist, während hingegen die gegenüberliegende Öffnung als Langloch 54b ausgebildet ist. Diese Ausgestaltung führt dazu, dass die Platine 51 im Bereich des Lochs 54a definiert festgelegt, an der gegenüberliegenden Seite hingegen gegenüber der Innenschale 30 verschiebbar, also schwimmend gelagert ist. Sollten sich im Falle von Temperaturschwankungen Platine 51 und Innenschale 30 unterschiedlich ausdehnen, können hierdurch Spannungen in der Platine 51, welche letztendlich zu Beschädigungen führen könnten, vermieden werden.

Eine thermisch optimierte Kopplung zwischen Lichtquelle 50 und Innenschale 30 kann hierbei dadurch erreicht werden, dass die in Figur 6 erkennbare Rückseite der Platine 51 mit mehreren oben bereits erwähnten Wärmeleitpads 55 versehen ist, die sich strahlen- bzw. fächerförmig ausgehend vom Zentrum der Platine 51 nach außen erstrecken und flächig auf der Oberseite des Bodenbereichs 31 der Innenschale 30 aufliegen. Diese Wärmeleitpads 55, welche acht elektrisch voneinander isolierte Segmente bilden, die gemeinsam vorzugsweise mindestens 80% bis 90% der Platinenfläche bedecken, verteilen die von dem mittig angeordneten LED-Leuchtmittel 52 ausgehende Wärme zunächst flächig über die Rückseite der Platine 51 und leiten diese auf den Bodenbereich 31 der Innenschale 30. Zur Verbesserung der Wärmeübertragung von Platine 51 zur Innenschale 30 sind bevorzugt geeignete thermisch leitende und elektrisch isolierende Interface-Materialen, wie beispielsweise Wärmeleitpaste oder Wärmeleitfolien vorgesehen.

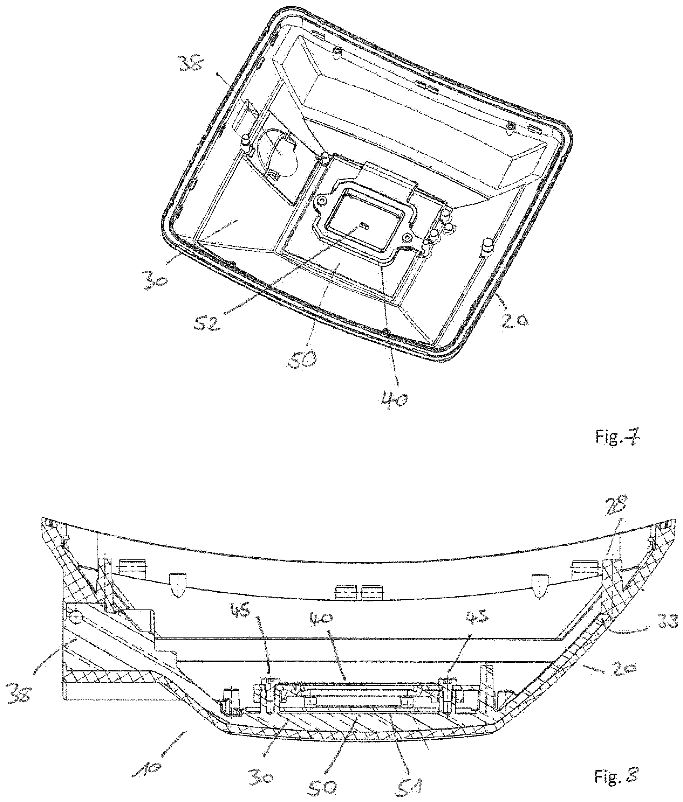

Die zuvor erwähnten Stege bzw. Stifte 32 dienen dabei zunächst lediglich einer korrekten Ausrichtung der LED-Platine 51 an der Oberseite der Innenschale 30. Die eigentliche Befestigung der Platine 51 erfolgt mithilfe eines in den Figuren 7 und 8 erkennbaren Klemmelements 40, welches einen Rahmen bildet, der umlaufend federnd auf die Oberseite der Platine 51 drückt. Die Befestigung des Klemmelements 40 an der Innenschale 30 erfolgt im dargestellten Ausführungsbeispiel über eine Schraubverbindung, wobei die bereits erwähnten Stege 32 nicht nur der Ausrichtung der Platine 51 dienen, sondern darüber hinaus auch Schraubkanäle bilden, in welche zwei mit dem Klemmelement 40 zusammenwirkende Schrauben eingreifen. Zusätzlich kann das Klemmelement 40 über weitere Arme oder Stege in das Gehäuse 10 eingehängt sein, wie dies in Figur 7 erkennbar ist.

Grundsätzlich wären auch andere Arten der Befestigung der Platine 51 in dem Gehäuse 10 denkbar, die dargestellte Lösung gewährleistet allerdings in vorteilhafter Weise, dass die Platine 51 großflächig auf die Oberseite des Bodenbereichs 31 der Innenschale 30 gedrückt wird und somit eine besonders gute Wärmeankopplung erzielt wird, wobei trotz allem das Auftreten von Spannungen im Fall von Temperaturschwankungen vermieden wird.

Ausgehend von dem Bodenbereich 31 der Innenschale wird dann primär über diese die Wärme verteilt, wobei dies allerdings auch für zumindest einen Teil des Wandbereichs des Gehäuses 10 gilt. Die Innenschale 30 erstreckt sich nämlich, wie die Schnittdarstellungen der Figuren 4 und 8 verdeutlichen, auch in den seitlichen Wandbereich 33 des Gehäuses 10, allerdings nicht ganz bis zur eigentlichen Lichtaustrittsöffnung 18. Die aus Kunststoff bestehende Außenschale 20 überragt im Wandbereich somit die Innenschale 30, was letztendlich zu einer formschlüssigen Verbindung beider Schalen 20, 30 miteinander führt. In dem der Lichtaustrittsöffnung 18 zugewandten Wandbereich kann hierbei die aus Kunststoff bestehende Außenschale 20 zusätzliche Strukturelemente 28 aufweisen, mit deren Hilfe dann eine Befestigung der lichtdurchlässigen Abdeckung sowie der optischen Elemente zur Beeinflussung des von der Lichtquelle 50 abgegebenen Lichts erfolgt.

Das Leuchtengehäuse 10 weist dabei an einer Seite des Wandbereichs 13 ein Lagergehäuse 38 auf, mit dessen Hilfe die schwenkbare Befestigung der Leuchte 100 an dem bereits erwähnten Tragarm 110 erfolgt. Dieses Lagergehäuse 38 bildet eine etwa zylinderartige Öffnung in dem Wandbereich 13 des Gehäuses 10, in welche ein entsprechender Endbereich 115 des Tragarms 110 eingreift, wobei mittels entsprechender Lager dann ein Schwenken des Leuchtengehäuses 10 um die Längsachse des Lagergehäuses 38 ermöglicht wird. Das Lagergehäuse 38 ist hierbei vorzugsweise integraler Bestandteil der Innenschale 30, was den Vorteil mit sich bringt, dass das Lagergehäuse 30 ebenso wie die gesamte Innenschale 30 aus Metall besteht und damit in der Lage ist, größere Kräfte aufzunehmen. Selbst für den Fall, dass das Gewicht der Leuchte 100 - beispielweise durch nicht näher dargestellte zusätzliche Anbauteile - erhöht wird, wird hierdurch eine dauerhaft zuverlässige und sichere schwenkbare Lagerung der Leuchte 100 an dem Tragarm 110 ermöglicht, ohne dass Verschleißerscheinungen zu befürchten sind.

Die Innenschale 30 als Teil des Leuchtengehäuses 10 dient somit vorteilhaft sowohl als thermische Senke für die Lichtquelle 10 als auch der schwenkbaren Befestigung des Leuchte 100 am Tragarm 110.

Die beschriebene doppellagige Ausgestaltung des Leuchtengehäuses 10 mit der Innenschale 30 und der Außenschale 20 führt nunmehr dazu, dass die von dem LED-Leuchtmittel 52 erzeugte Wärme effizient und sicher an die Umgebung abgeführt werden kann. Denn die primär mit der LED-Platine 51 gekoppelte Innenschale 30, deren Wärmeleitfähigkeit um ein Vielfaches (vorzugsweise zumindest etwa um den Faktor 100) größer ist als die Wärmeleitfähigkeit des Kunststoffmaterials der Außenschale 20, ermöglicht zunächst, dass die lokal produzierte Wärme großflächig, das heißt sowohl über den Bodenbereich 31 als auch über den Wandbereich 33 der Innenschale 30 verteilt wird. Dadurch, dass Außenschale 20 und Innenschale 30 flächig aneinander anliegen, erfolgt dann eine weitere Übertragung der Wärme von der Innenschale 30 auf die Außenschale 20 sowie abschließend über die Außenschale 20 ein endgültiges Ableiten der Wärme an die Umgebung, wobei sich nunmehr aufgrund der deutlich größeren Fläche die reduzierte Wärmeleitfähigkeit der Außenschale 20 nicht mehr negativ bemerkbar macht.

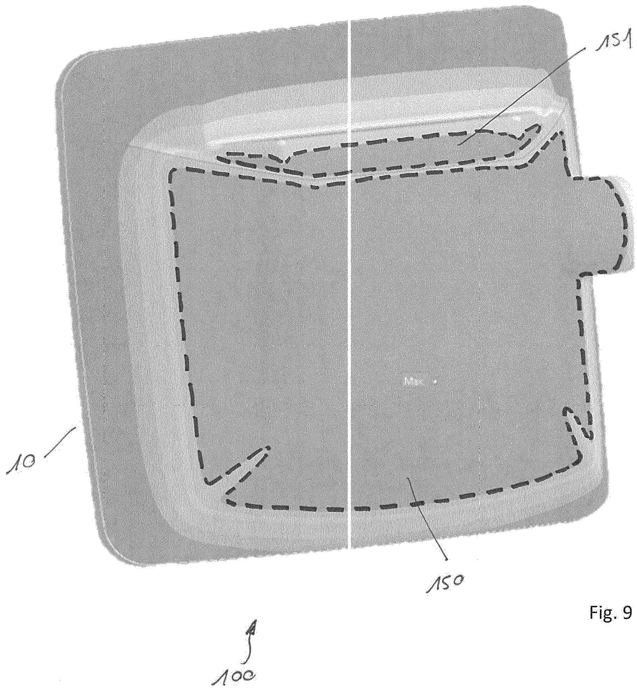

Genau genommen kann durch die Nutzung des Kunststoffmaterials für die Außenschale 20 sogar verhindert werden, dass sich an der Oberfläche der Leuchte in dem Bereich des LED-Leuchtmittels 52 ein Hotspot bildet. Stattdessen erwärmt sich die Außenfläche des Leuchtengehäuses 10 über den Bereich, der im Wesentlichen den Abmessungen der Innenschale 30 entspricht, nahezu gleichmäßig und homogen, wie die Darstellung in Figur 9 zeigt. Die hier gestrichelt eingerahmten beiden Bereiche 150 und 151 stellen diejenigen Bereiche der Gehäuseoberfläche dar, die während des Betriebs der Leuchte 100 eine erhöhte Temperatur aufweisen, wobei in den beiden Bereichen allerdings eine nahezu konstante Temperatur vorliegt. Die Ränder dieser Bereiche markieren gleichzeitig auch den Übergang zu dem Bereich, in dem der Wandbereich des Gehäuses 10 nur noch durch die Außenschale 20 gebildet ist, wobei erkennbar ist, dass ab dieser Grenze dann ein stetiger Temperaturabfall vorliegt. Über den vollständigen Bereich der Innenschale 30 hinweg hingegen wird die Wärme bei vergleichsweise niedriger Temperatur homogen an die Umgebung abgegeben.

Das vorteilhafte Zusammenwirken zwischen Außenschale 20 und Innenschale 30 des Gehäuses 10 wird hierbei dadurch gefördert, dass beide Schalen 20, 30 flächig und ohne störende Lufteinschlüsse aneinanderliegen. Besonders bevorzugt kann dies dadurch erreicht werden, dass in einem ersten Schritt die Innenschale 30 bereitgestellt und anschließend das die Außenschale 20 bildende Kunststoffmaterial aufgespritzt wird. Dies kann im Rahmen eines Spritzgussverfahrens erfolgen, bei dem also die zunächst z.B. als Druckgussteil hergestellte Innenschale 30 in die Spritzgussform eingelegt wird. Wie bereits erwähnt entsteht bei dieser Vorgehensweise keine stoffschlüssige Verbindung zwischen Außenschale 20 und Innenschale 30. Allerdings ist das vollflächige Anliegen beider Schalen 20, 30 zueinander gewährleistet, wobei aufgrund der Tatsache, dass - wie in den Schnittdarstellungen - erkennbar, die Außenschale 20 zumindest teilweise die Innenschale 30 übergreift, auch ein Formschluss zwischen beiden Komponenten gewährleistet ist.

Die oben beschriebene Vorgehensweise führt außerdem zu dem Vorteil, dass im Rahmen des Spritzgussverfahrens die Außenkonturen des Leuchtengehäuses 10 bildenden Bereiche mit hoher Präzision gefertigt werden können. Die Innenschale wiederum, die beispielsweise aus Aluminium oder Magnesium besteht, kann hingegen mit einer deutlich geringeren Präzision gefertigt werden, wobei darüber hinaus auch ein aufwendiges Nachbearbeiten entfällt.

Letztendlich wird also mithilfe der beschriebenen Lösung ein Leuchtengehäuse für medizinische, insbesondere für dentalmedizinische Zwecke zur Verfügung gestellt, welches in verhältnismäßig einfacher Weise hergestellt werden kann und in äußerst positiver Weise Wärme auch von sehr kleinen Lichtquellen ableitet.

## Patentansprüche

1. Medizinische Leuchte (100), insbesondere zahnärztliche Behandlungsleuchte für das Ausleuchten eines Operationsfelds, mit
• einer Lichtquelle (50), welche zumindest ein auf einer Platine (51) angeordnetes LED-Leuchtmittel (52) aufweist, sowie
• einem topfartigen Gehäuse (10) zur Aufnahme der Lichtquelle (50), welches eine zur Lichtabgabe vorgesehene Lichtaustrittsöffnung (18) bildet,
wobei das Gehäuse einen Bodenbereich (11) zur flächigen Lagerung der Platine (51) sowie einen sich von dem Bodenbereich (11) zur Lichtaustrittsöffnung umlaufend erstreckenden Wandbereich (13) aufweist,
**dadurch gekennzeichnet,**
**dass** das Gehäuse (10) durch eine aus Kunststoff bestehende Außenschale (20) sowie eine aus Metall bestehende, an der Innenseite der Außenschale (20) anliegende Innenschale (30) gebildet wird und die Platine (51) in flächigen Wärmekontakt mit der Innenschale (30) ist, welche sich über den Bodenbereich (11) in den Wandbereich (13) hinein erstreckt.

2. Medizinische Leuchte nach Anspruch 1,
**dadurch gekennzeichnet,**
**dass** die Außenschale (20) im Wandbereich (13) des Gehäuses (10) die Innenschale (30) überragt und sich bis zur Lichtaustrittsöffnung (18) erstreckt.

3. Medizinische Leuchte nach Anspruch 2,
**dadurch gekennzeichnet,**
**dass** die Außenschale (20) im der Lichtaustrittsöffnung (18) zugewandten Wandbereich (13) Befestigungsmittel (28) zur Halterung einer die Lichtaustrittsöffnung (18) schließenden lichtdurchlässigen Abdeckung und/oder optischer Elemente zur Beeinflussung des von der Lichtquelle (50) abgegebenen Lichts aufweist.

4. Medizinische Leuchte nach einem der vorherigen Ansprüche,
**dadurch gekennzeichnet,**
**dass** die Außenschale (20) ein oder mehrere Griffelemente (15) bildet.

5. Medizinische Leuchte nach einem der vorherigen Ansprüche,
**dadurch gekennzeichnet,**
**dass** die Innenschale (30) im Bodenbereich (11) Strukturelemente (32) zur definierten Positionierung der Platine (51) aufweist, wobei die Strukturelemente (32) insbesondere Stifte oder Stege bilden, welche in der Platine (51) ausgebildete Zentrierungsöffnungen (54) durchgreifen.

6. Medizinische Leuchte nach Anspruch 5,
**dadurch gekennzeichnet,**
**dass** die Platine (51) zwei Zentrierungsöffnungen (54) aufweist, wobei eine der Zentrierungsöffnungen (54a) einen auf die Außenkontur des Stifts oder Stegs angepassten Querschnitt aufweist und die andere Zentrierungsöffnung (54b) durch ein Langloch gebildet ist, welches eine Relativverschiebung zwischen Platine (51) und Innenschale (30) ermöglicht.

7. Medizinische Leuchte nach Anspruch 5 oder 6,
**dadurch gekennzeichnet,**
**dass** diese ferner ein rahmenartiges Klemmelement (40) aufweist, welches die Platine (51) federnd auf die Innenschale (30) drückt.

8. Medizinische Leuchte nach Anspruch 7,
**dadurch gekennzeichnet,**
**dass** das Klemmelement (40) mittels einer Schraubhalterung (45) an der Innenschale (30) befestigt ist, wobei vorzugsweise die Strukturelemente (32) Schraubkanäle zur Befestigung des Klemmelements (40) bilden.

9. Medizinische Leuchte nach einem der vorherigen Ansprüche,
**dadurch gekennzeichnet,**
**dass** das LED-Leuchtmittel (52) zentral auf der Platine angeordnet ist und etwa 1% der Fläche der Platine (51) beansprucht,
wobei das LED-Leuchtmittel (52) vorzugsweise durch zwei Reihenschaltungen von mehreren, insbesondere von jeweils drei LEDs gebildet ist.

10. Medizinische Leuchte nach einem der vorherigen Ansprüche,
**dadurch gekennzeichnet,**
**dass** die Platine (51) an ihrer dem LED-Leuchtmittel (52) gegenüberliegenden Rückseite Wärmeleitpads (55) aufweist, welche mindestens 80% der Platinenfläche einnehmen, wobei sich die Wärmeleitpads (55) vorzugsweise strahlen- bzw. fächerförmig ausgehend vom Zentrum der Platine (51) nach außen erstrecken.

11. Medizinische Leuchte nach Anspruch 10,
**dadurch gekennzeichnet,**
**dass** die Wärmeleitpads (55) elektrisch voneinander isolierte Bereiche bilden, die jeweils über Durchkontaktierungen mit Anschlüssen des LED-Leuchtmittels (52) gekoppelt sind.

12. Medizinische Leuchte nach einem der vorherigen Ansprüche,
**dadurch gekennzeichnet,**
**dass** die Innenschale (30) ein Lagergehäuse (38) zur Halterung der Leuchte (100) an einem Tragarm (115) aufweist und wobei insbesondere das Lagergehäuse (38) zur drehbaren Aufnahme des Tragarms (115) ausgebildet ist.

13. Medizinische Leuchte nach einem der vorherigen Ansprüche,
**dadurch gekennzeichnet,**
**dass** die Wärmeleitfähigkeit des Materials der Innenschale (30) etwa um den Faktor 100 größer ist als die Wärmeleitfähigkeit des Materials der Außenschale (20).

14. Medizinische Leuchte nach einem der vorherigen Ansprüche,
**dadurch gekennzeichnet,**
**dass** die Innenschale (30) aus Aluminium oder Magnesium besteht, wobei es sich insbesondere um ein Aluminium- oder Magnesiumdruckgussteil handelt.

15. Medizinische Leuchte nach einem der vorherigen Ansprüche,
**dadurch gekennzeichnet,**
**dass** die Außenschale (20) durch Aufspritzen von Kunststoffmaterial auf die Innenschale (30) gebildet ist, so dass die Außenschale (20) unmittelbar an der Innenschale (30) anliegt, wobei vorzugsweise die Außenschale (20) einen Randbereich (33) der Innenschale (30) zumindest teilweise übergreift.

## Claims

1. Medical lamp (100), in particular dental treatment lamp for illuminating an operating field, having
• a light source (50) that has at least one LED illuminant (52) arranged on a circuit board (51) and
• a pot-like housing (10) that serves to accommodate the light source (50) and forms a light exit opening (18) provided for light emission,
wherein the housing has a base region (11) for mounting of the circuit board (51) extensively and a wall region (13) that extends around the periphery from the base region (11) to the light exit opening,
**characterized**
**in that** the housing (10) is formed by a polymer outer shell (20) and a metal inner shell (30) adjacent to the inside of the outer shell (20), and the circuit board (51) is in extensive thermal contact with the inner shell (30), which extends over the base region (11) into the wall region (13).

2. Medical lamp according to Claim 1,
**characterized**
**in that** the outer shell (20) protrudes beyond the inner shell (30) in the wall region (13) of the housing (10) and extends to the light exit opening (18).

3. Medical lamp according to Claim 2,
**characterized**
**in that** in the wall region (13) facing the light exit opening (18), the outer shell (20) comprises fastening means (28) for mounting a light-transmitting cover that closes off the light exit opening (18) and/or optical elements for influencing the light emitted by the light source (50).

4. Medical lamp according to any of the preceding claims,
**characterized**
**in that** the outer shell (20) forms one or more gripping elements (15).

5. Medical lamp according to any of the preceding claims,
**characterized**
**in that** in the base region (11), the inner shell (30) comprises structural elements (32) for defined positioning of the circuit board (51), wherein the structural elements (32) in particular form pins or projections which engage in centring openings (54) formed in the circuit board (51).

6. Medical lamp according to Claim 5,
**characterized**
**in that** the circuit board (51) has two centring openings (54), wherein one of the centring openings (54a) has a cross section matched to the outer contour of the pin or projection, and the other centring opening (54b) is formed by a slot which allows a relative displacement between the circuit board (51) and the inner shell (30).

7. Medical lamp according to Claim 5 or 6,
**characterized**
**in that** said medical lamp further comprises a frame-like clamping element (40), which presses the circuit board (51) resiliently onto the inner shell (30).

8. Medical lamp according to Claim 7,
**characterized**
**in that** the clamping element (40) is fastened to the inner shell (30) by means of a screw holder (45), wherein the structural elements (32) preferably form screw channels for fastening the clamping element (40).

9. Medical lamp according to any of the preceding claims,
**characterized**
**in that** the LED illuminant (52) is located centrally on the circuit board and occupies about 1% of the area of the circuit board (51),
wherein the LED illuminant (52) is preferably formed by two series circuits of multiple LEDs, in particular three LEDs in each case.

10. Medical lamp according to any of the preceding claims,
**characterized**
**in that** the circuit board (51) has thermal pads (55), which occupy at least 80% of the circuit board area, on the rear side of said circuit board opposite the LED illuminant (52), wherein the thermal pads (55) preferably extend outwards in a radiating or fan-shaped manner starting from the centre of the circuit board (51).

11. Medical lamp according to Claim 10,
**characterized**
**in that** the thermal pads (55) form regions which are electrically insulated from each other and each of which are coupled by way of vias to terminals of the LED illuminant (52).

12. Medical lamp according to any of the preceding claims,
**characterized**
**in that** the inner shell (30) comprises a bearing housing (38) for mounting the lamp (100) on a support arm (115), wherein in particular the bearing housing (38) is designed to rotatably accommodate the support arm (115).

13. Medical lamp according to any of the preceding claims,
**characterized**
**in that** the thermal conductivity of the material of the inner shell (30) is greater than the thermal conductivity of the material of the outer shell (20) by about a factor of 100.

14. Medical lamp according to any of the preceding claims,
**characterized**
**in that** the inner shell (30) is made of aluminium or magnesium, in particular being an aluminium or magnesium die-cast part.

15. Medical lamp according to any of the preceding claims,
**characterized**
**in that** the outer shell (20) is formed by overmoulding polymer material onto the inner shell (30) such that the outer shell (20) directly abuts the inner shell (30), the outer shell (20) preferably engaging over an edge region (33) of the inner shell (30) at least in part.

## Revendications

1. Lampe médicale (100), en particulier lampe de traitement dentaire destinée à éclairer un champ opératoire, comprenant
• une source de lumière (50), laquelle possède au moins un moyen d'éclairage à LED (52) disposé sur une carte de circuit imprimé (51), et
• un boîtier en forme de pot (10) destiné à recevoir la source de lumière (50), lequel forme une ouverture de sortie de lumière (18) prévue pour l'émission de lumière, le boîtier possédant une zone de fond (11) destinée au montage en surface de la carte de circuit imprimé (51) ainsi qu'une zone de paroi (13) qui s'étend sur la circonférence depuis la zone de base (11) jusqu'à l'ouverture de sortie de lumière,
**caractérisée en ce**
**que** le boîtier (10) est formé par une coque extérieure (20) constituée de matière plastique ainsi que d'une coque intérieure (30) constituée de métal, qui repose contre le côté intérieur de la coque extérieure (20), et la carte de circuit imprimé (51) est en contact thermique sur toute la surface avec la coque intérieure (30), laquelle s'étend au-delà de la zone de fond (11) dans la zone de paroi (13).

2. Lampe médicale selon la revendication 1,
**caractérisée en ce**
**que** la coque extérieure (20) dépasse de la coque intérieure (30) dans la zone de paroi (13) du boîtier (10) et s'étend jusqu'à l'ouverture de sortie de lumière (18).

3. Lampe médicale selon la revendication 2,
**caractérisée en ce**
**que** la coque extérieure (20) possède, dans la zone de paroi (13) dirigée vers l'ouverture de sortie de lumière (18), des moyens de fixation (28) destinés à maintenir un couvercle transparent fermant l'ouverture de sortie de lumière (18) et/ou des éléments optiques destinés à influencer la lumière émise par la source de lumière (50).

4. Lampe médicale selon l'une des revendications précédentes,
**caractérisée en ce**
**que** la coque extérieure (20) forme un ou plusieurs éléments de préhension (15).

5. Lampe médicale selon l'une des revendications précédentes,
**caractérisée en ce**
**que** la coque intérieure (30) possède, dans la zone de fond (11), des éléments structuraux (32) servant au positionnement défini de la carte de circuit imprimé (51), les éléments structuraux (32) formant notamment des tiges ou des nervures qui traversent des ouvertures de centrage (54) formées dans la carte de circuit imprimé (51).

6. Lampe médicale selon la revendication 5,
**caractérisée en ce**
**que** la carte de circuit imprimé (51) possède deux ouvertures de centrage (54), l'une des ouvertures de centrage (54A) possédant une section transversale adaptée au contour extérieur de la tige ou de la nervure et l'autre ouverture de centrage (54B) étant formée par un trou oblong, lequel permet un décalage relatif entre la carte de circuit imprimé (51) et la coque intérieure (30).

7. Lampe médicale selon la revendication 5 ou 6, **caractérisée en ce**
**que** celle-ci possède en outre un élément de serrage (40) en forme de cadre, lequel presse élastiquement la carte de circuit imprimé (51) sur la coque intérieure (30).

8. Lampe médicale selon la revendication 7,
**caractérisée en ce**
**que** l'élément de serrage (40) est fixé à la coque intérieure (30) au moyen d'un élément de maintien à vis (45), les éléments structuraux (32) formant de préférence des canaux de vissage pour la fixation de l'élément de serrage (40).

9. Lampe médicale selon l'une des revendications précédentes,
**caractérisée en ce**
**que** le moyen d'éclairage à LED (52) est disposé au centre de la carte de circuit imprimé et occupe environ 1 % de la surface de la carte de circuit imprimé (51),
le moyen d'éclairage à LED (52) étant de préférence formé par deux circuits en série de plusieurs, en particulier respectivement de trois LED.

10. Lampe médicale selon l'une des revendications précédentes,
**caractérisée en ce**
**que** la carte à circuit imprimé (51) possède, sur sa face arrière opposée au moyen d'éclairage à LED (52), des pastilles thermoconductrices (55) qui occupent au moins 80 % de la surface de la carte à circuit imprimé, les pastilles thermoconductrices (55) s'étendant de préférence vers l'extérieur à partir du centre de la carte à circuit imprimé (51) en forme de faisceau ou d'éventail.

11. Lampe médicale selon la revendication 10,
**caractérisée en ce**
**que** les pastilles thermoconductrices (55) forment des régions électriquement isolées les unes des autres, qui sont respectivement couplées aux bornes du moyen d'éclairage à LED (52) par l'intermédiaire de trous métallisés.

12. Lampe médicale selon l'une des revendications précédentes,
**caractérisée en ce**
**que** la coque intérieure (30) possède un logement de palier (38) destiné à maintenir la lampe (100) sur un bras porteur (115), et le logement de palier (38) étant notamment configuré pour la réception en rotation du bras porteur (115).

13. Lampe médicale selon l'une des revendications précédentes,
**caractérisée en ce**
**que** la conductivité thermique du matériau de la coque intérieure (30) est supérieure à la conductivité thermique du matériau de la coque extérieure (20) d'un facteur d'environ 100.

14. Lampe médicale selon l'une des revendications précédentes,
**caractérisée en ce**
**que** la coque intérieure (30) est constituée d'aluminium ou de magnésium, celle-ci étant notamment une pièce coulée sous pression en aluminium ou en magnésium.

15. Lampe médicale selon l'une des revendications précédentes,
**caractérisée en ce**
**que** la coque extérieure (20) est formée par pulvérisation de matière plastique sur la coque intérieure (30), de sorte que la coque extérieure (20) repose directement contre la coque intérieure (30), la coque extérieure (20) recouvrant de préférence au moins partiellement une zone de bord (33) de la coque intérieure (30).
